# DEMANDE DE BREVET EUROPEEN

(11) **EP 4 837 526 A1**
(43) Date de publication de la demande: **11.11.2026**
(21) Numéro de dépôt: 26174802.4
(22) Date de dépôt: 27.04.2026
(51) Int. Cl.: A61B 17/17, A61F 2/46

(54) **SYSTÈME ET PROCÉDÉ POUR POSER UNE PROTHÈSE, NOTAMMENT SUR LA GLÈNE D'UNE SCAPULA**

(30) Priorité: 05.05.2025 FR 2504725
(71) Demandeur: FX Shoulder Solutions, 01440 Viriat (FR)
(72) Inventeur: MARTIN, Baptiste, 01440 VIRIAT (FR)
(74) Mandataire: LLR

(57) **Abrégé**

L'invention porte sur un système pour réaliser un fraisage pour la pose d'une prothèse sur un os qui comprend des moyens pour réaliser un modèle numérique de l'os, y compris le cartilage, pour déterminer un fraisage optimal de l'os, des moyens de fraisage et, des moyens pour visualiser le fraisage en temps réel afin notamment de déterminer en temps réel une position relative des moyens de fraisage et de l'os. L'invention porte aussi sur un procédé utilisant un tel système, notamment pour la pose d'une prothèse 20A sur la glène 2 d'une scapula 1.

## Description

### Domaine technique

La présente invention concerne le domaine des prothèses articulaires ; elle concerne notamment un système et un procédé pour implanter une prothèse d'articulation gléno-humérale d'une épaule, entre la scapula et l'humérus. Plus particulièrement, l'invention concerne un système et un procédé pour poser un implant articulaire se substituant à la glène.

La pose d'un implant de substitution à la glène est particulièrement délicate à réaliser. En effet, la scapula est en grande partie de faible épaisseur ; il n'est facile de poser un implant dans cette faible épaisseur. Il est donc important de préserver au maximum la masse osseuse entourant la glène pour y implanter efficacement cet implant.

### État de la technique

Préalablement à la pose de l'implant, une suppression du labrum et un fraisage de la glène est habituellement réalisé pour former un appui pour l'implant. Ce fraisage permet de retirer le cartilage et suffisamment d'os. L'os est arrasé jusqu'au saignement, pour l'aviver, afin que des cellules viennent coloniser l'implant et garantir son scellement. Selon un processus de l'art antérieur, le fraisage est réalisé par un opérateur, qui est typiquement un chirurgien, par itération, jusqu'à ce qu'il arrive au profil souhaité. Cependant, ce processus est essentiellement lié à l'expérience de l'opérateur et à sa perception, plus ou moins complète et précise, des caractéristiques de la glène qu'il est en train de fraiser. Le processus itératif est intrinsèquement long. De plus ce processus, moins précis, risque de retirer plus d'os que nécessaire alors qu'il faut en préserver un maximum dans une scapula dont le stock osseux est naturellement faible.

### Résumé de l'invention

Un but de l'invention est de proposer des moyens pour réaliser une implantation plus rapide et plus précise.

Selon un premier objet, l'invention enseigne un système pour réaliser un fraisage en prévision de la pose d'une pièce d'une prothèse sur un os qui comprend :
- des moyens pour réaliser un modèle numérique de l'os, y compris le cartilage ;
- des moyens pour déterminer de façon automatique un fraisage optimal de l'os ;
- des moyens pour former un premier repaire sur cet os ;
- des moyens de fraisage ;
- des moyens pour visualiser le fraisage en temps réel ; et,
- des moyens pour déterminer en temps réel une position relative des moyens de fraisage et de l'os.

Les moyens pour réaliser le modèle numérique peuvent comprendre des moyens d'imagerie, de préférence un scanner ou un IRM.

Les moyens pour déterminer le fraisage optimal peuvent comprendre des moyens de traitement numérique et de préférence des moyens d'apprentissage pour ces moyens numériques, lesdits moyens d'apprentissage comprenant de préférence des moyens d'intelligence artificielle.

Les moyens de fraisage peuvent comprendre un deuxième repère, ce deuxième repère comprenant de préférence un code graphique, de préférence du type QR-code.

Les moyens pour visualiser le fraisage en temps réel peuvent comprendre des moyens de réalité augmentée, ces moyens de réalité augmentée pouvant être du type casque ou lunettes.

Selon un deuxième objet, l'invention enseigne un procédé utilisant un système selon l'invention qui comprend des étapes pour :
- réaliser un modèle numérique de l'os, y compris le cartilage ; et
- déterminer de façon automatique un fraisage optimal, à partir de ce modèle numérique.

Un tel procédé peut en outre comprendre des étapes pour :
- former un premier repaire sur l'os ; et
- fraiser et visualiser le fraisage pour déterminer en temps réel une position relative des moyens de fraisage et de l'os.

Ce procédé peut être un procédé pour la pose d'une prothèse sur la glène d'une scapula.

### Brève description des figures

Des modes de réalisation et des variantes seront décrits ci-après, à titre d'exemples non limitatifs, avec référence aux dessins annexés dans lesquels :
[Fig. 1] illustre deux vues selon deux directions horizontales d'une scapula droite d'un être humain ;
[Fig. 2] illustre deux vues, selon une même direction de la glène de la scapula de la figure 1 ; et,
[Fig. 3] est une vue en coupe d'une prothèse inversée pour une articulation d'épaule pour la scapula de la figure 1.

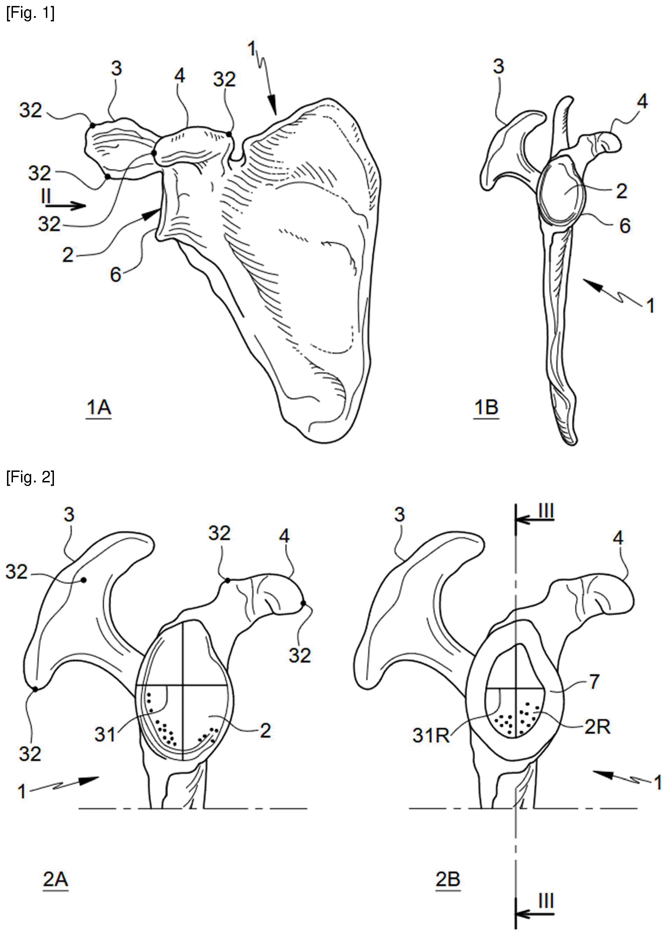

Les figures 1-3 sont toutes des vues en élévation, c'est-à-dire que le haut de la figure correspond au haut du corps de l'être humain, potentiellement le patient, dont la scapula est représentée, lorsque cet être humain se tient debout. L'expression « scapula droite » renvoie à la propre droite de cet être humain.

La figure 1 illustre la scapula 1 d'une épaule droite. La figure comprend deux vues 1A, 1B de la scapula. La vue 1A, à gauche de la figure 1, représente la scapula 1 de face ; la vue 1B, à droite de la figure 1, représente la scapula 1 de côté, la glène 2 faisant face à l'observateur. L'acromion 3 et la coracoïde 4 sont aussi visibles sur les vues de la figure 1.

La figure 2 illustre plus particulièrement la glène 2. Cette figure comprend deux vues 2A, 2B, prises selon la direction II de la figure 1A. A droite de la figure 2, la vue 2A illustre la glène 2 dans sa configuration initiale, avant fraisage ; A gauche de la figure 2, la vue 2B illustre la glène 2 après fraisage.

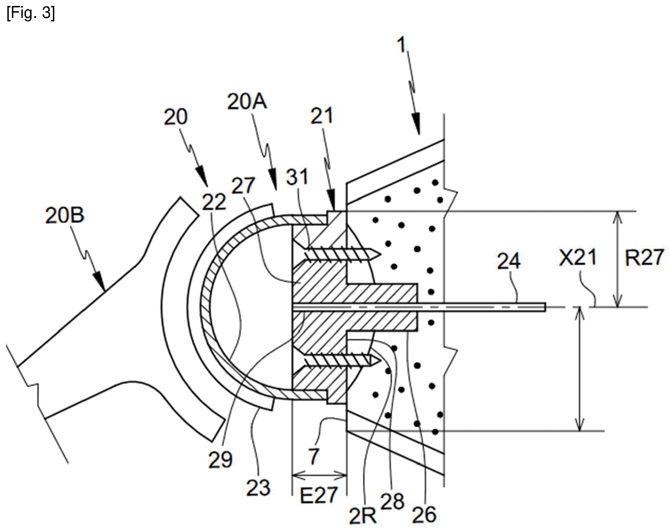

La glène 2 a une forme de cuvette, concave, bordée par un labrum périphérique annulaire 6. Le procédé selon l'invention comprend une phase de fraisage de la crête 6 pour former un appui 7 afin d'y disposer un insert 21 d'un implant glénoïdien d'une prothèse articulaire 20, de la façon illustrée à la figure 3. L'appui 7 a la forme d'un plateau annulaire autour de la partie résiduelle 2R de la glène 2. La prothèse n'est pas représentée à la figure 2.

La figure 3 illustre une coupe de la prothèse 20, selon le plan de coupe III-III de la vue 2B. La prothèse 20 comprend une partie scapulaire 20A et une partie humérale 20B. La partie scapulaire 20A est fixée à la scapula. La partie humérale 20B est fixée à l'humérus. Les deux parties 20A et 20B forment ensemble une articulation de type rotule, qui reconstitue la fonction articulaire de l'épaule, entre la scapula et l'humérus. La prothèse 20 est une prothèse inversée, c'est-à-dire que la partie scapulaire 20A a une forme convexe, alors que la partie humérale a une forme concave, les formes coopérant pour constituer une rotule.

La partie scapulaire 20A comprend l'insert 21, un bulbe 22 (ou glénosphère) hémisphérique, une cupule 23 et une broche 24. La figure 3 illustre schématiquement et en coupe la disposition de ces éléments 21-24, lorsque la prothèse 20 est en position fonctionnelle.

L'insert a une forme de champignon de révolution autour d'un axe d'insert X21 ; il comprend une tige 26 et une tête 27. La tête a la forme d'un disque de faible épaisseur E27 au regard de son rayon R27. La tige 26 s'étend axialement depuis une face interne 28 de la tête. L'insert 21 est percé d'un passage axial 29, prévu pour y recevoir la broche 24 et solidariser cette broche avec l'insert 21. Des vis 31 sont prévues pour fixer la tête 27 de sorte que la face interne 28 soit plaquée contre l'appui 7. Dans l'exemple illustré on utilise quatre vis, dont deux sont représentées à la figure 3.

Le bulbe 22 est fixé à la tête de l'insert, qui lui sert de potence. La cupule 23 vient recouvrir le bulbe et réduire les frottements entre la partie scapulaire 20A et la partie humérale 20B de l'articulation 20. typiquement le bulbe 22 a un diamètre d'environ vingt-quatre millimètres.

Dans un procédé selon l'invention de préparation de la phase de fraisage de l'appui 7, on réalise d'abord un modèle numérique de l'épaule du patient, en trois dimensions (3D). Ce modèle est réalisé à l'aide de moyens d'imagerie médicale. Ces moyens d'imagerie peuvent être du type scanner et/ou IRM. Ces moyens fournissent des points répartis en plusieurs images en deux dimensions (2D) ; les images 2D sont compilées pour former une image 3D constituée d'un nuage de points, ce nuage de point étant une accumulation de points des images 2D compilées. Le modèle d'épaule du patient est issu de l'image 3D ainsi formée. Le modèle distingue les différents constituants présents à l'image, notamment les cartilages et l'os de la scapula, notamment au niveau de la glène.

Dans ce procédé de préparation de la phase de fraisage, on utilise des moyens de traitement informatique pour définir automatiquement un fraisage optimal à réaliser. Ce fraisage doit former un appui 7 dépourvue de cartilage et l'os doit être suffisamment avivé pour assurer le scellement de l'insert 21, sans retirer trop d'os. Ces moyens de définition automatique d'un fraisage optimal sont avantageusement entrainés automatiquement, à partir de données issues de précédents fraisages, notamment réalisés de manière itérative comme expliqué plus haut. Il peut être aussi corrigé par l'opérateur, avant ou en cours de l'opération de fraisage et apprendre de ces corrections.

Pour les opérations de fraisage, on utilise une fraise. Cette fraise peut être portée et utilisée manuellement par l'opérateur. Le fraisage peut être automatisée et la manipulation de la fraise peut être confiée à un robot qui utilise des instructions générées par les moyens informatiques à partir du fraisage optimal défini précédemment. La fraise n'est pas représentée aux figures.

On définit aussi un premier repère 31, 32 (visible sur la figure 2) à la surface de la glène et on utilise des moyens de visualisation pour contrôler l'avancement de l'opération de fraisage, au regard du fraisage optimal prédéfini.

Notamment lorsque l'opérateur utilise une fraise manuelle, la fraise comprend un deuxième repère qui lui est propre, de sorte que les moyens de visualisation peuvent déterminer une position relative du premier repère 31, 32 et du deuxième repère. Les moyens de visualisation peuvent avantageusement comprendre des moyens de réalité augmentée, par exemple un casque ou des lunettes de réalité augmentée, de sorte que l'opérateur peut visualiser en temps réel la progression du fraisage. Le deuxième repère peut être un ou plusieurs codes graphique porté par la fraise. Ce code graphique peut être du type QR-Code ou similaire.
On va maintenant décrire un premier type de premier repaire, en référence à la figure 2.

Comme illustré à la vue 2A de la figure 2, le premier type peut être un premier repère 31 peut être tracée par l'opérateur en travers de la glène 2. Dans l'exemple illustré, ce premier repère a une forme de croix, lorsque la glène est vue de face. Lors de l'opération de fraisage, la matière est enlevée avec les portions du premier repère 31 qui y étaient tracées. A la fin du fraisage, comme illustré à la vue 2A, seule une partie résiduelle 31A du premier repère 31 est encore visible sur la partie résiduelle 2R de la glène. L'effacement progressif du premier repère 31 permet de déterminer l'avancement du fraisage. Le fraisage peut être réalisé en-dessous du centre de la croix, de préférence à une distance sensiblement égale à douze millimètres du centre de la croix.

On va maintenant décrire un deuxième type de premier repaire, en référence à aux figures 1 et 2.

Comme illustré sur les vues 1A et 2A, le deuxième type de premier repère est constitués de plusieurs points caractéristiques 32, répartis sur la scapula du patient. Dans l'exemple illustré, il y a deux points 32 sur l'acromion 3 et deux autres points 32 sur la coracoïde 4. Ces points sont choisis en nombre suffisant pour former un repère visible par les moyens de visualisation durant toute la phase de fraisage. Les points du repère 32 peuvent comprendre des points choisis de façon automatisée par les moyens informatique dans le nuage de point du modèle ; ils peuvent aussi comprendre des points marqués physiquement par l'opérateur sur la scapula 1.

Bien entendu, les deux types de premier repère peuvent être utilisés simultanément.

Bien sûr, l'invention n'est pas limitée aux exemples qui viennent d'être décrits. Au contraire, l'invention est définie par les revendications qui suivent.

Il apparaîtra en effet à l'homme de l'art que diverses modifications peuvent être apportées aux modes de réalisation décrits ci-dessus, à la lumière de l'enseignement qui vient de lui être divulgué.

## Revendications

1. Système pour réaliser un fraisage en prévision de la pose d'une pièce (21) d'une prothèse (20A) sur un os (1) **caractérisé en ce qu'**il comprend :
des moyens pour réaliser un modèle numérique dudit os, y compris le cartilage ;
des moyens pour déterminer de façon automatique un fraisage optimal de l'os ;
des moyens pour former un premier repaire (31,32) sur ledit os ;
des moyens de fraisage ;
des moyens pour visualiser le fraisage en temps réel ; et,
des moyens pour déterminer en temps réel une position relative des moyens de fraisage et de l'os.

2. Système selon la revendication 1, **caractérisé en ce que** les moyens pour réaliser le modèle numérique comprennent des moyens d'imagerie, de préférence un scanner ou un IRM.

3. Système selon la revendication 1 ou 2, **caractérisé en ce que** les moyens pour déterminer le fraisage optimal comprennent des moyens de traitement numérique et de préférence des moyens d'apprentissage pour lesdits moyens numériques, lesdits moyens d'apprentissage comprenant de préférence des moyens d'intelligence artificielle.

4. Système selon l'une des revendications 1 à 3, **caractérisé en ce que** les moyens de fraisage comprennent un deuxième repère, ledit deuxième repère comprenant de préférence un code graphique, de préférence du type QR-code.

5. Système selon l'une des revendications 1 à 4, **caractérisé en ce que** les moyens pour visualiser comprennent des moyens de réalité augmentée, lesdits moyens de réalité augmentée pouvant être du type casque ou lunettes.

6. Procédé **caractérisé en ce qu'**il comprend des étapes pour :
- fournir un système pour réaliser un modèle numérique d'un os (1) en prévision de la pose d'une pièce (21) d'une prothèse (20A) sur ledit os, ledit appareil comprenant des moyens pour réaliser un modèle numérique dudit os, y compris le cartilage et des moyens pour déterminer de façon automatique un fraisage optimal de l'os ;
- réaliser un modèle numérique de l'os, y compris le cartilage ; et,
- déterminer de façon automatique un fraisage optimal, à partir dudit modèle numérique.

7. Procédé selon la revendication 6, **caractérisé en ce que** l'étape pour réaliser le modèle numérique comprend l'usage de moyens d'imagerie, de préférence un scanner ou un IRM.

8. Procédé selon la revendication 6 ou 7, **caractérisé en ce que** l'étape pour déterminer de façon automatique un fraisage optimal, à partir dudit modèle numérique, comprend l'usage de moyens de traitement numérique et de préférence de moyens d'apprentissage pour lesdits moyens numériques, lesdits moyens d'apprentissage comprenant de préférence des moyens d'intelligence artificielle.

9. Procédé selon l'une des revendication 6 à 8, **caractérisé en ce qu'**il est utilisé en prévision de la pose d'un insert (21) d'une prothèse (20A) d'une glène (2) d'une scapula (1).
